# EUROPEAN PATENT SPECIFICATION

(11) **EP 1 067 907 B1**
(45) Date of publication and mention of the grant of the patent: **07.01.2004**
(21) Application number: 99915876.9
(22) Date of filing: 07.04.1999
(51) Int. Cl.: A61K 9/08

(54) **OCULAR IRRIGATING SOLUTION**
AUGENSPÜLLÖSUNG
SOLUTION D'IRRIGATION OCULAIRE

(30) Priority: 07.04.1998 GB 9807491
(43) Date of publication of application: 17.01.2001
(73) Proprietor: UNIVERSITY OF BRISTOL, Clifton Bristol BS8 1TH (GB)
(72) Inventor: ARMITAGE, William John, Bristol, Avon BS9 3PT (GB); YAGOUBI, Mohamed I., Uni. Bristol, Opthalmology, Bristol, Avon BS1 2LX (GB)
(74) Representative: Nash, David Allan
(86) International application number: PCT/GB1999/001066
(87) International publication number: WO 1999/051204

(56) References cited:
- EP-A- 0 435 797
- EP-A- 0 778 021
- DE-A- 19 626 479
- FR-A- 2 602 677
- GB-A- 2 064 320
- US-A- 4 725 586

## Description

This invention relates to aqueous solutions for use in surgical procedures, and is particularly concerned with an ophthalmic irrigating solution useful for irrigating the human eye during surgery.

A description of the problems associated with surgical procedures, especially surgical procedures performed on the eye, and the historical development of tissue irrigating solutions may be found in EP-A-0076658.

The stated object of EP-A-0076658 is to provide a stable sterile ophthalmic irrigating solution which, in addition to correct electrolyte balance, provides factors necessary for continued metabolism in the endothelial cells, maintenance of the fluid transport pump system, and consequential maintenance of proper corneal thickness and clarity. This problem is stated to be achieved in EP-A-0076658 by providing a two-part solution system which includes a basic solution and an acidic solution which are individually stable and which, on mixing, form an ocular solution which contains the necessary factors to maintain endothelial cell integrity and corneal thickness during ocular surgery. The combined solution contains the necessary ions in a bicarbonate-phosphate buffer as well as oxidised glutathione and dextrose (d-glueose), the latter being present as an energy source.

There are problems associated with the solution system of EP-A-0076658. Firstly, such a system is relatively expensive because two separate solutions must be prepared and separately sterilised; this problem is not easy to overcome because certain of the ingredients of the system, particularly the oxidised glutathione and the glucose, are heat-labile and cannot therefore be sterilised by an autoclaving procedure as required by various regulatory authorities for solutions exceeding about 500ml in volume which are to be used in surgical procedures. As a consequence, the two-part system of EP-A-0076658 is prepared, in practice, such that the non-labile components are present in the solution which contains the majority of the fluid which will form the final ocular solution, which is then bottled and autoclaved. The labile components are contained in the other solution of relatively small volume (below the threshold above which autoclaving is required) which may be sterilised by a filtration technique.

A second problem with the solution system of EP-A-0076658 is that its two-part nature can potentially lead to errors in forming the final ocular solution, a procedure which is normally conducted in a hospital.

US 4 725 586 discloses an ophtalmic solution, comprising chondroitine sulfate and 2-mercaptoethanol as essential components.

US-A-5 328 701 discloses a irrigation solution for use during eye surgery which contains a balanced amount of compatible electrolytes and at least one compound from the citric acid cycle.

HEPES has been proposed, in the 1980 article "Intraocular irrigating and replacement fluid", M.V. Graham et al, Trans. Ophthal. Soc. U.K. (1980) 100, p282-285, as a buffer for an intraocular irrigating solution. However, the 1983 article, "A Comparison of HEPES and Bicarbonate Buffered Intraocular Irrigating Solutions: Effects on Endothelial Function in Human and Rabbit Corneas", by Dayle H. Geroski et al, J. Toxicol - Cut & Ocular Toxicol 1(4), 299-309, (1982-83) concludes that HEPES is toxic to endothelial Na⁺K⁺ATPase and questions the prudence of using HEPES buffer in intraocular irrigating solutions.

It would be an advantage to provide a stable ophthalmic irrigating solution as a single solution capable of being sterilised by autoclaving.

It has now been found that a solution which is effective as an ophthalmic irrigating solution can be formed which does not require the glutathione ingredient previously believed to be essential, but does include a specific buffer to ensure that the proper pH is maintained prior to and during use.

The present invention relates to an aqueous occular irrigating solution as defined in claim 11 and the use of the solution as defined in claim 1.

The organic buffer preferably maintains the solution at a pH in the range 7.2 to 7.8 to match the physiological pH of 7.4.

Highly preferred as the organic buffer are the zwitterionic amino acids, such as N-2-[hydroxyethyl]piperazine-N'-[2-ethanesulfonic acid], commonly referred to as HEPES, which has a pKa of 7.55 at 25°C. Other organic buffers in this family are N,N-bis[2-hydroxyethyl]-2-aminoethanesulfonic acid (BES), pKa=7.1; 3-[N-morpholino]propanesulfonic acid (MOPS), pKa=7.2 at 25°C; N-tris[hydroxymethyl]methyl-2-aminoethanesulfonic acid (TES), pKa=7.4 at 25°C; N-[2-hydroxyethyl]-piperazine-N'-[3-propanesulfonic acid] (EPPS), pKa=8.0 at 25°C; N-tris[hydroxymethyl]methylglycine (TRICINE), pKa=8.1 at 25°C.

The organic buffer should be present in the solution in an amount sufficient to buffer the solution over the duration of the surgical procedure. In practice, this means that the concentration of the buffer should be about 10 to 50 mmol/l.

The bicarbonate source is normally sodium bicarbonate. The bicarbonate source is preferably present in the solution to give a bicarbonate concentration of about 10 to 50 mmol/l, preferably from 15 to 25 mmol/ml to maintain the fluid pump system in the endothelium of the eye.

The ocular irrigating solutions of the present invention are free from glutathione, which has previously been considered essential for effective performance.

Hitherto it has been considered essential for ocular irrigating solutions to contain an energy source which is purportedly required as a substrate for the various metabolic pathways taking place in the cornea. It has now surprisingly been discovered that ocular irrigating solutions which are free from an energy source (such as glucose) are capable of supporting endothelial function and maintaining corneal thickness as well as solutions containing the energy source. Thus, irrigation solutions of the invention do not contain an energy source. This is of particular significance so far as glucose is concerned which tends to degrade at physiological pH over extended time periods.

The solution of the invention preferably also contains other electrolytes necessary to maintain physiological function, such as Na⁺, K⁺, Ca²⁺, and Cl⁻, but not Mg²⁺, which can lead to the formation of magnesium precipitates in some circumstances. These should be present at concentrations which will permit continued cellular integrity and metabolism.
Typically, these electrolytes are present in the following concentrations:

| | |
|---|---|
| Na⁺ | 130 - 180 mmol/l |
| K⁺ | 3 - 10 mmol/l |
| Ca²⁺ | up to 5 mmol/l |
| Cl⁻ | 130 - 210 mmol/l |

Preferably the concentration of Ca²⁺ is at least 0.05 mmol/l, and preferably no more than 0.1 mmol/l.

Moreover, the osmolality should be between approximately 250 - 350 mosmol/kg, preferably 290 - 320 mosmol/kg, to maintain osmotic stability of the cells.

Also normally present in the solution will be a source of phosphate ions, although primarily not for buffering purposes, as in EP-A-00766598, but for normal physiological function. The approximate concentration of phosphate in the solution is normally about 1 mmol/l.

The solution of the invention may be prepared by mixing the components together in aqueous solution, in the desired proportions. It may then be bottled and autoclaved in the normal manner.

One advantage of the invention is that it may be autoclaved without any deleterious effect. For this reason, components which would degrade to a significant extent under the chosen autoclave conditions should be excluded or reduced in amount to a point at which degradation is minimal. Typical autoclave conditions are 121°C for 15 minutes or 134°C for 3 min.

The ocular solution of the invention should preferably be free from nutrients of the type normally present in tissue culture media, namely: amino acids, vitamins, hormones, proteins, growth factors, lipids, nucleosides, minerals.

The solution of the invention may be used in a method of surgery performed on the human eye to replace fluid loss during the operation and to maintain corneal function. Thus according to another aspect of the invention, there is provided an aqueous solution, comprising a source of bicarbonate ions and a physiologically acceptable organic buffer which is an organic zwitterionic buffer having a buffering capacity within the range pH 6.8 to 8.0, for use in a surgical method, preferably a surgical method performed on the eye.

The invention will now be illustrated by reference to the following Example and drawings in which:

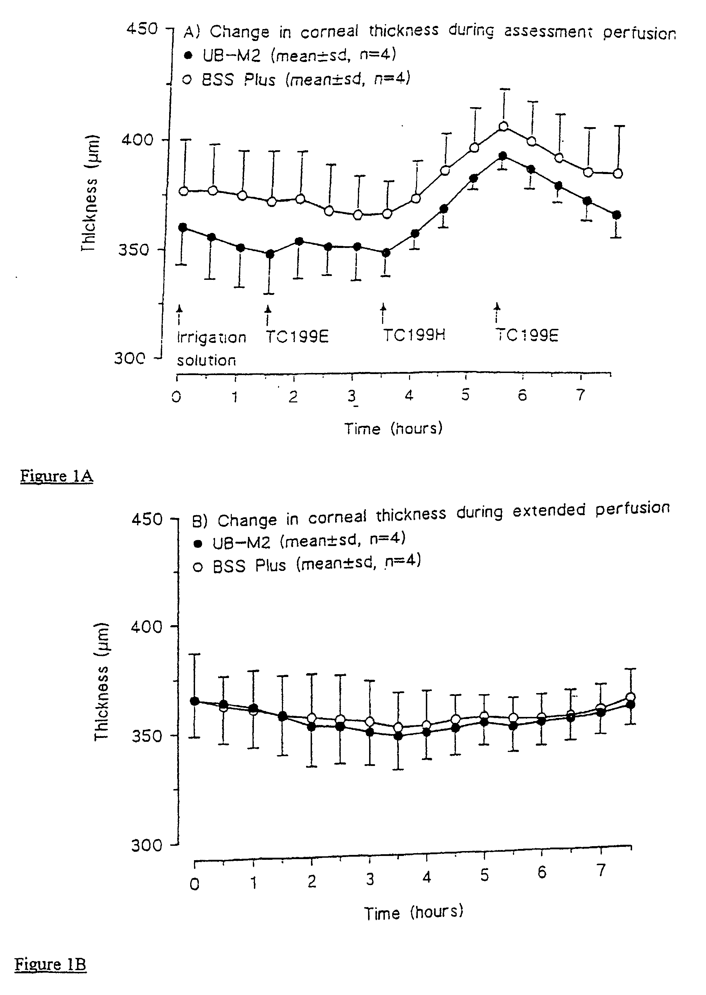

Figure 1A shows the change in corneal thickness during assessment perfusion following 90 minutes exposure to the "UB-M2" solution in accordance with the invention and "BSS Plus";
Figure 1B shows the change in corneal thickness during perfusion with a solution in accordance with the invention "UB-M2" solution and with "BSS Plus".

### Example 1

A prior art irrigating solution and an irrigating solution in accordance with the invention were tested in a masked laboratory experiment to evaluate their effectiveness. BSS Plus (which is in accordance with EP-A-0076658) was obtained as a two part system and made up as directed. The composition of these solutions along with those of aqueous humour and BSS are shown in Table 1.

**Table 1**

| | Aqueous humour | BSS | BSS Plus | Invention |
|---|---|---|---|---|
| Na⁺ (mM) | 162.9 | 144.0 | 160.0 | 137.2 |
| K⁺ (mM) | 2.2-3.9 | 10.0 | 5.0 | 5.4 |
| Ca²⁺ (mM) | 1.8 | 4.3 | 1.0 | 0.075 |
| Mg²⁺ (mM) | 1.1 | 3.2 | 1. 0 | - |
| Cl⁻ (mM) | 131.6 | 127.2 | 130.0 | 121.2 |
| HCO₃⁻ (mM) | 20.2 | - | 25.0 | 20.0 |
| HPO₄²⁻ (mM) | 0.6 | - | 3.0 | 0.8 |
| SO₄²⁻ (mM) | - | - | - | - |
| Acetate (mM) | - | 28.6 | - | - |
| Citrate (mM) | - | 5.8 | - | - |
| Lactate (mM) | 2.5-4.5 | - | - | - |
| Glucose (mM) | 2.7-3.7 | - | 5.0 | - |
| Glutathione | 1.9 µM | - | 0.3 mM | - |
| HEPES (mM) | - | - | - | 20.0 |
| Osmolality (mosmol/kg) | 304 | 302 | 305 | 320 |
| pH (20°C) | 7.4 | 7.3 | 7.4 | 7.4 |

Corneas obtained from New Zealand White rabbits (3-4 kg) after an intravenous overdose of pentobarbitone sodium were secured on support rings and perfused as described in J. Physiol 1972; 221: 29-41, "The metabolic basis to the fluid pump in the cornea", Dikstein S. and Maurice DM. The paired corneas from each rabbit were randomly allocated, one to BSS Plus and one to the invention. The allocation was unknown to the person performing the experiment. The epithelial surface was covered with silicone oil to prevent changes in corneal thickness owing to evaporation.

The endothelial surface was perfused at 2.5 ml/h, a pressure of 15 cm H₂O and 35°C. During the first 90 minutes of perfusion, corneas were exposed to the intraocular irrigation solution. This was followed by a further 6 hours of perfusion during which endothelial function was assessed.

Corneal thickness was measured with an ultrasonic pachymeter (DGH Technologies, Inc), every 30 minutes. The silicone oil was removed briefly to allow the measurements to be made. Each measurement was the mean of readings taken at four different sites of the central cornea.

Changes in corneal thickness during perfusion for 90 minutes with the irrigation solutions are shown in Figure 1A.

Corneal hydration and, thus, thickness are controlled by the endothelium through a pump leak mechanism. Removal of bicarbonate ions from the perfusate suppresses endothelial pump function and causes corneal swelling, although inhibition of the pump is not complete unless CO₂ is also removed from the perfusate. Pump function can be restored and the swelling reversed by returning bicarbonate to the perfusate.

After the 90 minute perfusion with one of the irrigation solutions, endothelial function was, therefore, assessed during a further 6 hours of perfusion with Tissue Culture Medium 199 (TC199). The first 2 hours of perfusion were with TC199 with Earle's salts (Sigma, M3769). This solution contained sodium bicarbonate (26 mmol/l), and should have supported endothelial pump function. Two hours of perfusion with TC199 with Hanks' salts (Sigma, M3274) then followed. This solution did not contain bicarbonate ions and, thus, should have caused corneal swelling, although the solution was not CO₂ free. For the final 2 hours, perfusion with TC199 Earle's was restored and, providing that the endothelium was undamaged, corneas should have thinned. Neither of the TC199 solutions contained phenol red, and their measured osmolalities (Roebling osmometer) were 290 and 288 mosmol/kg, respectively, for TC199 with Earle's salts and TC199 with Hanks' salts.

Rates of change in corneal thickness both during perfusion with the irrigation solutions and during the three parts of the assessment perfusion were determined by regression analysis. Comparisons were made between groups by t-tests at the 5% level of significance. The results obtained are shown in Table 2 and are also illustrated graphically in Figure 1A.

**Table 2**

| | | Rate of change in corneal thickness (µm/h)^{b} | | |
|---|---|---|---|---|
| Irrigation solution^{a} | Initial 90 min exposure to irrigation solution | TC199 Earle's 0-2 h | TC199 Hank's^{c} 2-4 h | TC199 Earle's 4-6 h |
| BSS Plus | -5.1(4.3) | +0.02(4.2) | +16.1(1.9) | -13.1(5.3) |
| Invention | -8.4(3.5) | +1.4 (4.7) | +17.7(2.0) | -13.4(3.7) |

| | | | | |
|---|---|---|---|---|
| ^{a}Corneas were perfused for 90 minutes with an irrigation solution before the assessment perfusion with TC199. | | | | |
| ^{b}regression coefficient (SD), n=4: + indicates swelling, - indicates thinning. | | | | |
| ^{c}TC199 Hanks' does not contain HCO₃⁻. | | | | |

There were no differences at the 5% level of significance in rates of change in thickness between corneas exposed to BSS Plus and those exposed to the irrigating solution in accordance with the invention at any stage of the perfusion.

### Example 2

An ocular irrigating solution in accordance with the invention was made up as in Example 1.

Corneas were dissected, mounted on support rings and perfused as in Example 1 except that the corneas were perfused continuously for a period of 7.5 hours with either BSS Plus or the invention. Paired corneas, from a single rabbit, were perfused, one with BSS Plus and the other with the invention. The allocation of corneas to each solution was randomized and masked from the person performing the perfusion. Regression analysis showed no overall change (at the 5% level of significance) in thickness during the course of the perfusion nor was corneal thickness influenced by the type of irrigation solution (see Figure 1B).

In conclusion, Examples 1 and 2 demonstrate that the invention supports endothelial function at least as well as BSS Plus, despite the absence of components, such as glucose and glutathione, that are considered essential constituents of BSS Plus.

## Claims

1. The use of an aqueous solution consisting essentially of a source of bicarbonate ions, a physiologically acceptable organic buffer which is an organic zwitterionic buffer having a buffering capacity within the range pH 6.8 to 8.0, and optionally a source of phosphate ions and/or a source of electrolytes necessary to maintain in the physiological function selected from Na⁺, K⁺, Ca²⁺ and Cl⁻, in the manufacture of an ocular irrigating solution for irrigating the eye during surgery.

2. A use according to claim 1, wherein the organic buffer maintains the solution at a pH in the range 7.2 to 7.8.

3. A use according to claim 1 or 2, wherein the organic buffer is a zwitterionic amino acid.

4. A use according to claim 3, wherein the organic buffer is N-2-[hydroxyethyl]piperazine-N'-[2-ethanesulfonic acid].

5. A use according to any preceding claim, wherein the concentration of the buffer is from 10 to 50 mmol/l.

6. A use according to any preceding claim, wherein the bicarbonate source is sodium bicarbonate.

7. A use according to claim 6, wherein the bicarbonate source is preferably present in the solution to give a bicarbonate concentration of about 10 to 50 mmol/l.

8. A use according to any preceding claim wherein the solution does not contain glucose, or any other energy source which tends to degrade at physiological pH over extended time periods.

9. A use according to any preceding claim wherein the solution has been sterilised by an autoclaving procedure.

10. A use according to any preceding claim wherein the ocular irrigating solution replaces fluid loss during surgery and maintains corneal function.

11. An aqueous ocular irrigating solution for irrigating of eye during surgery consisting essentially of a source of bicarbonate ions, a physiologically acceptable organic buffer which is an organic zwitterionic buffer having a buffering capacity within the range pH 6.8 to 8.0, sources of electrolytes necessary to maintain physiological function selected from Na⁺, K⁺, Ca²⁺ and Cl⁻ and optionally a source of phosphate ions, and wherein the bicarbonate source is present in the solution to give a bicarbonate concentration of from 10 to 50 mmol/l.

12. An ocular irrigating solution according to claim 11 wherein the organic buffer maintains the solution at a pH in the range 7.2 to 7.8.

13. An ocular irrigating solution according to claim 11 or claim 12 wherein the organic buffer is a zwitterionic amino acid.

14. An ocular irrigating solution according to any one of claims 11 to 13 wherein the organic buffer is N-2-[hydroxyethyl]piperazine-N'-[2-ethanesulfonic acid].

15. An ocular irrigating solution according to any one of claims 11 to 14 wherein the concentration of the buffer is from 10 to 50 mmol/l.

16. An ocular irrigating solution according to any one of claims 11 to 15 wherein the bicarbonate source is sodium bicarbonate.

17. An ocular irrigating solution according to any one of claims 11 to 16 wherein the bicarbonate source is present in the solution to give a bicarbonate concentration of from 15 to 25 mmol/l.

18. An ocular irrigating solution according to any one of claims 11 to 17 which does not contain glucose, or any other energy source which tends to degrade at physiological pH over extended time periods.

19. An ocular irrigating solution according to any one of claims 11 to 18 having been sterilised by an autoclaving procedure.

## Patentansprüche

1. Verwendung einer wässrigen Lösung, bestehend im Wesentlichen aus einer Quelle für Bicarbonationen, einem physiologisch akzeptablen organischen Puffer, welches ein organischer Zwitterionen-Puffer mit einer Pufferkapazität im Bereich von pH 6,8 bis 8,0 ist, und wahlfrei einer Quelle für Phosphationen und/oder einer Quelle für Elektrolyten, welche für die Aufrechterhaltung der physiologischen Funktion notwendig sind, ausgewählt aus Na⁺, K⁺, Ca²⁺, und Cl⁻, zur Herstellung einer Augentropfenlösung zum Benetzen des Auges beim Operieren.

2. Verwendung nach Anspruch 1, wobei der organische Puffer die Lösung bei einem pH im Bereich von 7, 2 bis 7, 8 hält.

3. Verwendung nach Anspruch 1 oder 2, wobei der organische Puffer eine zwitterionische Aminosäure ist.

4. Verwendung nach Anspruch 3, wobei der organische Puffer N-2-[Hydroxyethyl]piperazin-N'-[2-ethansulfonsäure] ist.

5. Verwendung nach irgendeinem vorhergehenden Anspruch, wobei die Konzentration des Puffers 10 bis 50 mMol/l ist.

6. Verwendung nach irgendeinem vorhergehenden Anspruch, wobei die Bicarbonatquelle Natriumbicarbonat ist.

7. Verwendung nach Anspruch 6, wobei die Bicarbonatquelle bevorzugt in einer Lösung zugegen ist, welche eine Bicarbonatkonzentration von etwa 10 bis 50 mMol/l ergibt.

8. Verwendung nach irgendeinem vorhergehenden Anspruch, wobei die Lösung keine Glukose enthält oder eine andere Energiequelle, die dazu neigt, den physiologischen pH bei längeren Zeiträumen abzubauen.

9. Verwendung nach irgendeinem vorhergehenden Anspruch, wobei die Lösung durch Autoklavieren sterilisiert ist.

10. Verwendung nach irgendeinem vorhergehenden Anspruch, wobei die Augentropfenlösung den Flüssigkeitsverlust während einer Operation ersetzt und die Hornhautfunktion erhält.

11. Wässrige Augentropfenlösung zum Benetzen des Auges während einer Operation, bestehend im Wesentlichen aus einer Quelle für Bicarbonationen, einem physiologisch akzeptablen organischen Puffer, der ein organischer Zwitterionenpuffer mit einer Pufferkapazität im Bereich von 6,8 bis 8,0 pH ist, Quellen für Elektrolyten zur Aufrechterhaltung der physiologischen Funktion, ausgewählt aus Na⁺, K⁺, Ca²⁺, und Cl⁻, und wahlfrei eine Quelle für Phosphationen, wobei die Bicarbonatquelle in der Lösung zugegen ist und eine Bicarbonatkonzentration von 10 bis 50 mmol/l ergibt.

12. Augentropfenlösung nach Anspruch 11, wobei der organische Puffer die Lösung bei einem pH im Bereich von 7,2 bis 7,8 hält.

13. Augentropfenlösung nach Anspruch 11 oder 12, wobei der organische Puffer eine zwitterionische Aminosäure ist.

14. Augentropfenlösung nach irgendeinem der Ansprüche 11 bis 13, wobei der organische Puffer N-2-[Hydroxyethyl]piperazin-N'-[2-ethansulfonsäure] ist.

15. Augentropfenlösung nach irgendeinem der Ansprüche 11 bis 14, wobei die Konzentration des Puffers 10 bis 50 mMol/L ist.

16. Augentropfenlösung nach irgendeinem der Ansprüche 11 bis 15, wobei die Bicarbonatquelle Natriumbicarbonat ist.

17. Augentropfenlösung nach irgendeinem der Ansprüche 11 bis 16, wobei die Bicarbonatquelle in der Lösung zugegen ist und eine Bicarbonatkonzentration von 15 bis 25 mMol/L ergibt.

18. Augentropfenlösung nach irgendeinem der Ansprüche 11 bis 17, die keine Glucose enthält oder irgendeine andere Energiequelle, die dazu neigt, den physiologischen pH über längere Zeiträume abzubauen.

19. Augentropfenlösung nach irgendeinem der Ansprüche 11 bis 18, die durch Autoklavieren sterilisiert ist.

## Revendications

1. Utilisation d'une solution aqueuse consistant essentiellement en une source d'ions bicarbonates, un tampon organique physiologiquement acceptable qui est un tampon zwitterionique organique ayant une capacité tampon dans le domaine de pH 6,8 à 8,0, et éventuellement une source d'ions phosphate et/ou une source d'électrolytes nécessaires pour maintenir la fonction physiologique choisies parmi Na⁺, K⁺, Ca²⁺ et Cl⁻, dans la production d'une solution d'irrigation oculaire pour irriguer l'oeil pendant une chirurgie.

2. Utilisation selon la revendication 1, où le tampon organique maintient la solution à un pH dans le domaine de 7,2 à 7,8.

3. Utilisation selon la revendication 1 ou 2, où le tampon organique est un acide aminé zwitterionique.

4. Utilisation selon la revendication 3, où le tampon organique est l'acide N-2-[hydroxyéthyl]pipérazine-N'-[2-éthanesulfonique].

5. Utilisation selon l'une quelconque des revendications précédentes, où la concentration du tampon est de 10 à 50 mmol/l.

6. Utilisation selon l'une quelconque des revendications précédentes, où la source de bicarbonate est le bicarbonate de sodium.

7. Utilisation selon la revendication 6, où la source de bicarbonate est de préférence présente dans la solution pour donner une concentration de bicarbonate d'environ 10 à 50 mmol/l.

8. Utilisation selon l'une quelconque des revendications précédentes, où la solution ne contient pas de glucose, ou toute autre source d'énergie qui a tendance à se dégrader au pH physiologique pendant des durées prolongées.

9. Utilisation selon l'une quelconque des revendications précédentes, où la solution a été stérilisée par un processus d'autoclavage.

10. Utilisation selon l'une quelconque des revendications précédentes, où la solution d'irrigation oculaire remplace les pertes de fluide pendant une chirurgie et maintient la fonction cornéenne.

11. Solution aqueuse d'irrigation oculaire pour irriguer l'oeil pendant une chirurgie consistant essentiellement en une source d'ions bicarbonates, un tampon organique physiologiquement acceptable qui est un tampon zwitterionique organique ayant une capacité tampon dans le domaine de pH 6,8 à 8,0, des sources d'électrolytes nécessaires pour maintenir la fonction physiologique choisies parmi Na⁺, K⁺, Ca²⁺ et Cl⁻ et éventuellement une source d'ions phosphates, et où la source de bicarbonate est présente dans la solution pour donner une concentration de bicarbonate de 10 à 50 mmol/l.

12. Solution d'irrigation oculaire selon la revendication 11, où le tampon organique maintient la solution à un pH dans le domaine de 7,2 à 7,8.

13. Solution d'irrigation oculaire selon la revendication 11 ou la revendication 12, où le tampon organique est un acide aminé zwitterionique.

14. Solution d'irrigation oculaire selon l'une quelconque des revendications 11 à 13, où le tampon organique est l'acide N-2-[hydroxyéthyl]pipérazine-N'-[2-éthanesulfonique].

15. Solution d'irrigation oculaire selon l'une quelconque des revendications 11 à 14, où la concentration du tampon est de 10 à 50 mmol/l.

16. Solution d'irrigation oculaire selon l'une quelconque des revendications 11 à 15, où la source de bicarbonate est le bicarbonate de sodium.

17. Solution d'irrigation oculaire selon l'une quelconque des revendications 11 à 16, où la source de bicarbonate est présente dans la solution pour donner une concentration de bicarbonate de 15 à 25 mmol/l.

18. Solution d'irrigation oculaire selon l'une quelconque des revendications 11 à 17, qui ne contient pas de glucose ou toute autre source d'énergie qui a tendance à se dégrader au pH physiologique pendant une durée prolongée.

19. Solution d'irrigation oculaire selon l'une quelconque des revendications 11 à 18, qui a été stérilisée par un processus d'autoclavage.
